# EUROPEAN PATENT SPECIFICATION

(11) **EP 1 597 398 B1**
(45) Date of publication and mention of the grant of the patent: **23.04.2008**
(21) Application number: 04715423.2
(22) Date of filing: 27.02.2004
(51) Int. Cl.: C12Q 1/68, A61K 31/00, C07D 417/04

(54) **METHOD OF DIAGNOSTIC OF MASTOCYTOSIS**
DIAGNOSTISCHES VERFAHREN DER MASTOZYTOSE
METHODE DE DIAGNOSTIC DE LA MASTOCYTOSE

(30) Priority: 27.02.2003 US 449861 P
(43) Date of publication of application: 23.11.2005
(73) Proprietor: AB Science, 75008 Paris (FR)
(72) Inventor: KINET, Jean-Pierre, Lexington, MA 02421 (US); MOUSSY, Alain, F-75006 Paris (FR)
(74) Representative: Ahner, Francis
(86) International application number: PCT/IB2004/000907
(87) International publication number: WO 2004/076693

(56) References cited:
- WO-A-01/47517
- WO-A-02/080925
- WO-A-03/002105
- WO-A-03/002109
- WO-A-03/002114
- WO-A-03/003006
- WO-A-20/04014903
- NAGATA HIROSHI ET AL: "Identification of a point mutation in the catalytic domain of the protooncogene c-kit in peripheral blood mononuclear cells of patients who have mastocytosis with an associated hematologic disorder" PROCEEDINGS OF THE NATIONAL ACADEMY OF SCIENCES OF THE UNITED STATES, vol. 92, no. 23, 1995, pages 10560-10564, XP002286457 1995 ISSN: 0027-8424
- ZERMATI, YAEL ET AL: "Effect of tyrosine kinase inhibitor STI571 on the kinase activity of wild-type and various mutated c - kit receptors found i mast cell neoplasms" ONCOGENE (2003), 22(5), 660-664, 2003, XP002286458
- FEGER FREDERIC ET AL: "Kit and c-kit mutations in mastocytosis: A short overview with special reference to novel molecular and diagnostic concepts" INTERNATIONAL ARCHIVES OF ALLERGY AND IMMUNOLOGY, vol. 127, no. 2, February 2002 (2002-02), pages 110-114, XP009032820 ISSN: 1018-2438
- LONGLEY B J ET AL: "NEW APPROACHES TO THERAPY FOR MASTOCYTOSIS A CASE FOR TREATMENT WITH KIT KINASE INHIBITORS" HEMATOLOGY - ONCOLOGY CLINICS OF NORTH AMERICA, W.B. SAUNDERS, US, vol. 14, no. 3, June 2000 (2000-06), pages 689-695, XP008010967 ISSN: 0889-8588

## Description

The present invention relates to the diagnosis of mastocytosis comprising a reverse transcription of the RNA sample from skin.

Mastocytosis are a very heterogeneous group of disorders characterized by an abnormal accumulation of mast cells in different tissues, mainly in the skin and the bone marrow, but also in spleen, liver, lymph nodes, and the gastrointestinal tract, depending on the nature of the disease. They can affect humans of either sex at any age. Neoplasms of mast cells (MC) can be acute or chronic. Acute mast cell neoplasms are designated as MC leukemia. Chronic mast cell neoplasms may be localized or generalized. Cutaneous mastocytosis is the commonest localized neoplasm and is often found in children in which it often remits and never relapses. Mastocytosis are usually acquired diseases, but some rare familial cases have been described.

With regard to the extreme heterogeneity of mast cell neoplasms, it is important to classify these diseases. One of the most used classification is the one by Metcalfe (Metcalfe, J Invest Dermatol. 96: 2S-4S, 1991) that distinguishes four categories of mastocytosis :

**The category I** is composed by two sub-categories (IA and IB). Category IA is made by diseases in which mast cell infiltration is strictly localized to the skin. This category represents the most frequent form of the disease and includes : i) urticaria pigmentosa, the most common form of cutaneous mastocytosis, particularly encountered in children, ii) diffuse cutaneous mastocytosis, iii) solitary mastocytoma and iv) some rare subtypes like bullous, erythrodermic and teleangiectatic mastocytosis. These forms are characterized by their excellent prognosis with spontaneous remissions in children and a very indolent course in adults. Long term survival of this form of disease is generally comparable to that of the normal population and the translation into another form of mastocytosis is rare. Category IB is represented by indolent systemic disease (SM) with or without cutaneous involvement. These forms are much more usual in adults than in children. The course of the disease is often indolent, but sometimes signs of aggressive or malignant mastocytosis can occur, leading to progressive impaired organ function.

**The category II** includes mastocytosis with an associated hematological disorder, such as a myeloproliferative or myelodysplastic syndrome, or acute leukemia. These malignant mastocytosis does not usually involve the skin. The progression of the disease depends generally on the type of associated hematological disorder that conditiones the prognosis.

**The category III** is represented by aggressive systemic mastocytosis in which massive infiltration of multiple organs by abnormal mast cells is common. In patients who pursue this kind of aggressive clinical course, peripheral blood features suggestive of a myeloproliferative disorder are more prominent. The progression of the disease can be very rapid, similar to acute leukemia, or some patients can show a longer survival time.

Finally, **the category IV** of mastocytosis includes the mast cell leukemia, characterized by the presence of circulating mast cells and mast cell progenitors representing more than 10% of the white blood cells. This entity represents probably the rarest type of leukemia in humans, and has a very poor prognosis, similar to the rapidly progressing variant of malignant mastocytosis. Mast cell leukemia can occur either *de novo* or as the terminal phase of urticaria pigmentosa or systemic mastocytosis.

Since categories II and III do not differ prognostically, the classification of Metcalfe can be further simplified as shown in Table I, according to the recommendations of Homy et al (Horny et al, Am J Surg Pathol. 22: 1132-40, 1998).

**Table I**

| **Localized (category I)** | | **Generalized (categories II, III, IV)** | |
|---|---|---|---|
| *Cutaneous mastocytosis* | | *Systemic mastocytosis (with or without cutaneous involvement)* | |
| | Solitary mastocytoma | | Indolent |
| | Urticaria pigmentosa | | Aggressive |
| | | *Mast cell leukemia* | |

Mast cells are characterized by their heterogeneity, not only regarding tissue location and structure but also at the functional and histochemical levels (Aldenborg and Enerback., Histochem. J. 26: 587-96, 1994 ; Bradding et al. J Immunol. 155: 297-307, 1995 ; Irani et al, J Immunol. 147: 247-53, 1991 ; Miller et al, Curr Opin Immunol. 1: 637-42, 1989 and Welle et al, J Leukoc Biol. 61: 233-45, 1997). Differentiation, survival and proliferation of MC depend greatly on SCF (Torrey et al, 1990). The receptor for SCF is c-kit, encoded by the protooncogene c-kit; it belongs to type III receptor tyrosine kinase subfamily (Baghestanian et al, 1996). Numerous studies have been performed regarding the neoplastic mechanism of mastocytosis, searching for genetic abnormalities of c-kit (mutation, deletion). The existence of such abnormalities was suggested because they were previously found in rodent or human leukemic MC lines. In human mastocytosis, mutations of c-kit have been described in vivo in various forms of mastocytosis (cutaneous mastocytosis, systemic indolent or systemic aggressive mastocytosis). Among the mutations found, the most common is the activating mutation Asp to Val at codon 816. In addition, one report has described a mutation in the juxtamembrane domain of c-kit (Val to Gly at codon 560) in human mastocytosis (Valent et al, 1994). Furthermore, Longley et al (Pauls et al, 1999) have showed that the point mutations in position 816.

As of today, the clinical suspicion of mastocytosis is confirmed by histologic examination, especially of skin and bone marrow. Stains such as tuoluidine blue can be used to identify mast cells by staining their metachromatic granules. Also, the chloroacetate-esterase reaction can complete staining. In addition, immunocytochemistry for tryptase is useful to confirm the nature of the cellular infiltrate. Finally, the diagnostic can be helped by the use of the immunophenotyping of MC in bone marrow aspirate. Indeed, normal as well as mastocytosis-related mast cells strongly express CD117 antigen (Arber et al, Hum Pathol. 29: 498-504, 1998 ; Escribano et al, Cytometry. 30: 98-102, 1997), and some antigens not found on normal MC can be aberrantly expressed by neoplastic mast cells, such a CD2, CD25 and CD35 (Escribano et al Blood. 91: 2731-6, 1998, Ormerod et al, British Journal of Dermatology. 122: 737-44, 1990). Other findings have shown that the CD69 activation antigen is over-expressed on MC from patients with systemic mastocytosis, as compared to normal mast cells (Diaz-Agustin et al, Br J Haematol. 106: 400-5,1999).

Biochemical determination of mast cell mediators can also help to diagnosis of mastocytosis: histamine level in blood and urine, metabolites of prostaglandin D2 and of histamine in the urine are increased in most cases of SM, as well as the level of tryptase in blood (Hogan and Schwartz, Methods 13: 43-52, 1997 ∼ Van Gysel et al, J Am Acad Dermatol. 35: 556-8, 1996 ∼ Morrow et al, J Invest Dermatol. 104: 937-40, 1995 ; Marone et al, Chem Immunol. 62: 1-21, 1995). However, with these tests, some false positive (allergy) or false negative (mastocytosis without mediator release) may exist.

A number of studies have been performed to elucidate whether mutations of c-kit are associated with different clinical forms of mast cell diseases. Indeed, mutations of c-kit have been described *in vivo* in various forms of mastocytosis (cutaneous mastocytosis, systemic indolent or systemic aggressive mastocytosis). Among the mutations found, the there are the Asp to Val at codon 816 and juxtamembrane mutation.

Asp816Val mutation occurs in an early progenitor cell, and not in mature mast cells since it is carried by myelomonocytic cells, T cells, and B cells in addition to MC. The same activating point mutations in codon 816 of the c-kit gene have been described not only in patients with isolated mastocytosis but also in mastocytosis with an associated Hematological disorder, such as a myeloproliferative or myelodysplastic syndrome, or acute leukemia (Boissan and Arock, Leukoc Biol. 67: 135-48,2000).

More recently, we have proposed to use standard molecular biology techniques based on PCR in our patent application WO 03/002114 for precisely determining the activating mutation in a given patient. Probes and primers have been designed so as to be specific to such mutations analysis. We also described methods for identifying non-toxic specific c-kit inhibitors that are either active on SCF activated c-kit or on constitutively activated c-kit, notably on the 816 mutant c-kit, in our application WO 03/003006.

We now have discovered that 60 % of patients suspected to be afflicted with different forms of mastocytosis bear a mutation at the 816 position of c-kit. This observation has been possible by performing of a genotyping study on about 200 patients.

Unexpectedly, we also observed that diagnostic methods using bone marrow as sample (Proc. Natl. Acad. Sci. USA, 1995, vol. 92, pages 10560-10564) are not accurate and predictable enough to classify patients falling either in i) the 816 bearing mutation category or ii) the non-816 bearing mutation category On the contrary, methods based on skin sample have revealed accurate for that purpose.

Therefore, the invention provides a tailored treatment to patients belonging either to i) or ii) category allowing the administration of a relevant c-kit inhibitor. This is particularly useful since c-kit inhibitors may be active on 816-mutated c-kit or on c-kit wild or other forms of c-kit but not on both. The choice of the appropriate inhibitor is of great importance considering the inefficacy and the potential side effects of improperly administered c-kit inhibitors.

### Description

The invention relates to a method for diagnosing an individual suspected to be afflicted with category I, II, III and IV mastocytosis or symptoms associated with mast cells proliferation comprising a) reverse transcription of the RNA from skin sample using oligo dT primers and random primers, b) amplifying the cDNA obtained in step c) using primers:
- U2 (5' GGATGACGAGTTGGCCCTAGA 3') (SEQ ID No 1) and
- L1 (5' GTAGAACTTAGAATCGACCGGCA 3') (SEQ ID No 2),
and c) detecting the presence or the absence of a mutation of the c-kit c-DNA at position 816 of C-KIT.

In other words, it is aimed at a method comprising the use of SEQ ID No 1 and SEQ ID No 2 for amplifying and detecting specifically the presence or absence of the cDNA corresponding to the '816' mutation of C-KIT from cDNAs obtained from reversed transcribed skin total RNA sample. The following primers can also be used:

| | |
|---|---|
| (sens) | 5' ATCCTCCTTACTCATGGTCGGATC 3'(SEQ ID No 5) |
| (antisens) | 5' CGACCGGCATTCCAGGATAG 3' (SEQ ID No 6) |

It will be understood that the invention is aimed at these primers as well as any primers displaying non significant differences in the sequence frame or length.

Utility of the invention will further ensue from the detailed description below.

### Example 1: Mutation assessment in patients

### Material and Method

### RNA extractions

A section of frozen skin at - 80°C is poured in 500 µl RLT media (+ 5 µl of *β*mercaptoéthanol), which correspond to the buffer of the QIAGEN Rneasy Mini kit. It is grinded in a T25 basic ultra-turrax homogenizer and an ultra-turrax axis of 0.8 cm (ref B35333 de Fisher Bioblock Scientific). The grinded skin section is then frozen before RNA extraction.

Before each grinding, the ultra-turrax rod is rinsed twice in two 50 ml tubes containing non autoclaved and non DEPC treated milli Q water. The rod is then rinsed and cleaned with 250 ml ethanol 70% during 5 minutes. Elle est ensuite déposée dans un incubateur à 37°C pendant 45 minutes (étape de séchage) et remontée juste avant l'extraction suivante. Further steps are necessary to ensure no RNA degradation during grinding.

For the following tests a negative control is used. Two skin sections are extracted successively from the patient and are checked for the presence of the valine mutation at position 816 of the KIT receptor.

RNA is extracted from the grinded skin tumor with the QIAGEN (Rneasy Mini) kit. Each sample is thawed. The extraction is performed with a minimum of 4 samples to minimize risks of RNA degradation. 983 µl of milli Q water is added to 500 µl of RLT buffer and 17 µl of proteinase K (ref 19133 QIAGEN). We incubate 10 min at 55°C and centrifuge 3 min at 10 000 g at room temperature. The supernatant is mixed with 750 ml absolute ethanol. 700 µl of supernatant is run on a Rneasy minispin column and centrifuged at room temperature during 15 s at 8000 g. The remaining supernatant is run in the same column. We clean with 350 µl RW buffer. After centrifugation at 8000 q during 15 s, 10 µl of DNAse and 70 µl of RDD buffer is poured in the column (RNAse free DNAse set : ref 79254 de QIAGEN SA).

DNAse reacts during 15 minutes at room temperature. Then, 350 µl of RW1 buffer are added on the column to rinse (centrifugation of 15 sec at 8000 g).

The column is rinsed with RPE buffer then centrifuged 2 minutes à 8000 g to eliminate the RPE buffer. The RNA is then washed out elué with 30 µl of DEPC treated water and kept frozen at-80°C.

### Reverse transcriptase step

20 ng of RNA are used for the synthesis of the cDNA. We use the ProStar™ first strand RT-PCR kit of Stratagene. Then final volume of the reverse transcriptase reaction is 25 µl.

In a final volume of 17.5 µl containing 200 ng of RNA, we add 1.5 µl of oligo dT and 1.5 µl of random primers. The reaction mixture is incubated 5 min at 65°C then 10 minutes at room temperature. The synthesis of the first strand is performed after adding 2.5µl of = first strand 10X buffer, 0.5µl RNAse block ribonuclease inhibitor, 1µl of dNTP 100 mM, 0.5µl of reverse transcriptase StrataScript ™ and it is incubated during 1 hour at 42°C

### Amplification of the c-kit gene

2.5µl of the reverse transcriptase reaction is used for amplification. The amplification program is as follows:

| | |
|---|---|
| 94°C10min | |
| | |
| 94°C 15 sec | |
| 55°C 15 sec | 35 cycles |
| 72°C 30 sec | |
| | |
| 72°C 7 min | |
| 4°C 15h | |

Primers are U2 et L1:
U2 : 5' GGATGACGAGTTGGCCCTAGA 3'(SEQ ID No 1)
L1 : 5' GTAGAACTTAGAATCGACCGGCA 3'(SEQ ID No 2)

In case the quantity of amplified cDNA is not enough for sequencing, a nested-PCR is used using the above program and the following primers:
U2217 : 5' CCCAACCAAGGCCGACAAAAGGA 3'(SEQ ID No 3)
L2722 : 5' TCCCAGCAAGTCTTCATTATGTC 3'(SEQ ID No 4)

### Purification of PCR products

This step is performed using the GENECLEAN III® kit.

### Sequencing

U2 and L1 primers are used for the sequencing reaction. The following primers can also be used:

| | |
|---|---|
| (sens) | 5' ATCCTCCTTACTCATGGTCGGATC 3'(SEQ ID No 5) |
| (antisens) | 5' CGACCGGCATTCCAGGATAG 3'(SEQ ID No 6) |

## Claims

1. A method for diagnosing an individual suspected to be afflicted with category I, II, III and IV mastocytosis or symptoms associated with mast cells proliferation comprising a) reverse transcription of the RNA sample from skin using oligo dT primers and random primers, b) amplifying the cDNA obtained in step a) using primers :
U2 : 5' GGATGACGAGTTGGCCCTAGA 3'(SEQ ID No 1)
L1 : 5' GTAGAACTTAGAATCGACCGGCA 3'(SEQ ID No 2)
or
5' ATCCTCCTTACTCATGGTCGGATC 3'(SEQ ID No 5)
5' CGACCGGCATTCCAGGATAG 3'(SEQ ID No 6)
c) detecting the presence or the absence of a mutation of the c-kit c-DNA corresponding to position 816 of C-KIT.

## Patentansprüche

1. Verfahren zum Diagnostizieren eines individuums, bei welchem vermutet wird, dass es an Mastozytose der Kategorie I, II, II und IV oder Symptomen, die mit einer Proliferation von Mastzellen verbunden sind, leidet, umfassend: a) Umkehrtranskription der RNA-Probe aus Haut unter Verwendung von oligo dT-Primern und Zufallsprimern, b) Amplifizieren der in Schritt a) erhaltenen cDNA unter Verwendung der Primer:
U2 : 5' GGATGACGAGTTGGCCCTAGA 3' (SQ ID No. 1)
L1 : 5' GTAGAACTTAGAATCGACCGGCA 3' (SEQ ID No. 2)
oder
5' ATCCTCCTTACTCATGGTCGGATC 3' (SEQ ID No. 5)
5' CGACCGGCATTCCAGGATAG 3' (SEQ ID No. 6),
c) Detektieren des Vorhandenseins oder des Fehlens einer Mutation der c-kit-cDNA, welche Position 816 von C-KIT entspricht.

## Revendications

1. Procédé de diagnostic d'un individu suspecté d'être affecté d'une mastocytose de catégorie I, II, III ou IV ou de symptômes associés à une prolifération de mastocytes, comprenant a) la transcription inverse de l'ARN de l'échantillon de peau en utilisant des amorces oligo dT et des amorces aléatoires, b) l'amplification de l'ADNc obtenu dans l'étape a) en utilisant les amorces :
U2 : 5' GGATGACGAGTTGGCCCTAGA 3' (SEQ ID N° 1)
L1 : 5' GTAGAACTTAGAATCGACCGGCA 3' (SEQ ID N° 2)
ou
5' ATCCTCCTTACTCATGGTCGGATC 3' (SEQ ID N° 5)
5' CGACCGGCATTCCAGGATAG 3' (SEQ ID N° 6)
c) la détection de la présence ou de l'absence d'une mutation de l'ADNc de c-kit correspondant à la position 816 de C-KIT.


### SEQUENCE LISTING

<110> AB SCIENCE
<120> Tailored treatment suitable for different forms of mastocytosis
<130> D21041
<150> 60/449,861
   <151> 2003-02-27
<160> 6
<170> PatentIn version 3.2
<210> 1
   <211> 21
   <212> DNA
   <213> Artificial
<220>
   <223> U2 Primer
<400> 1
   ggatgacgag ttggccctag a 21
<210> 2
   <211> 23
   <212> DNA
   <213> Artificial
<220>
   <223> L1 Primer
<400> 2
   gtagaactta gaatcgaccg gca 23
<210> 3
   <211> 23
   <212> DNA
   <213> Artificial
<220>
   <223> U2217 Primer
<400> 3
   cccaaccaag gccgacaaaa gga 23
<210> 4
   <211> 23
   <212> DNA
   <213> Artificial
<220>
   <223> L2722 Primer
<400> 4
   tcccagcaag tcttcattat gtc 23
<210> 5
   <211> 24
   <212> DNA
   <213> Artificial
<220>
   <223> Sense Primer
<400> 5
   atcctcctta ctcatggtcg gatc 24
<210> 6
   <211> 20
   <212> DNA
   <213> Artificial
<220>
   <223> Antisense Primer
<400> 6
   cgaccggcat tccaggatag 20